# EUROPEAN PATENT APPLICATION

(11) **EP 2 777 685 A1**
(43) Date of publication of application: **17.09.2014**
(21) Application number: 12846838.6
(22) Date of filing: 06.11.2012
(51) Int. Cl.: A61J 3/00, B65B 1/30

(54) **DRUG PACKAGING DEVICE**

(30) Priority: 09.11.2011 JP 2011245906
(71) Applicant: Takazono Technology Incorporated, Osaka 573-0128 (JP)
(72) Inventor: MORIKAWA, Yasuyuki, Hirakata-shi Osaka 573-0128 (JP)
(74) Representative: Isarpatent
(86) International application number: PCT/JP2012/078692
(87) International publication number: WO 2013/069621

(57) **Abstract**

A medicine dispensing and packing apparatus includes a powder supplying portion, a plurality of powder dividing portions, a dispensing and packing portion, a control portion, and an input portion. The input portion includes an input screen. In a case where a number of packets larger than a maximum number of divisions by which one powder dividing portion can divide powder and a dividing portion use instruction to perform dispensing and packing using the plurality of powder dividing portions are inputted to the control portion by a user's operation on the input screen, the control portion makes allocations of the inputted number of packets to the powder dividing portions within a range not exceeding the maximum number of divisions, and controls the powder supplying portion, the plurality of powder dividing portions, and the dispensing and packing portion on a basis of the allocations.

## Description

The present application claims priority to Japanese Patent Application No. 2011-245906, the contents of which are incorporated by reference herein.

### FIELD

The present invention relates to a medicine dispensing and packing apparatus that can dispense and pack powder.

### BACKGROUND

A conventionally known medicine dispensing and packing apparatus of this type includes: a powder supplying portion that supplies powder; a plurality of powder dividing portions that divide the powder supplied from the powder supplying portion, into a plurality of portions by a predetermined dose; a dispensing and packing portion that dispenses and packs each dose of the powder divided by each of the powder dividing portions; a control portion that controls the powder supplying portion, the powder dividing portions, and the dispensing and packing portion; and an input portion through which the number of packets is inputted to the control portion (see, for example, Patent Literature 1 given below).

In such a medicine dispensing and packing apparatus, when the number of packets is inputted through a keyboard as the input portion, the control portion controls the powder supplying portion, the powder dividing portions, and the dispensing and packing portion to perform dispensing and packing, on the basis of the inputted contents.

Meanwhile, limitations on the number of days of prescription have been relaxed in Japan. Consequently, for example, in a prescription for a patient who needs to be on long-term medication, it has become possible to order the long-term medication at a time. When the long-term medication is ordered at a time in this way, the number of packets is larger accordingly. Hence, a number of packets that exceeds the maximum number of divisions by which one powder dividing portion can divide powder may be ordered depending on an ordered period of medication.

In the case where a number of packets that exceeds the maximum number of divisions is ordered in this way, the conventional medicine dispensing and packing apparatus performs dispensing and packing by dividing such a large number of packets into some divisional units. Specifically, in the conventional medicine dispensing and packing apparatus, when a large number of packets is inputted through the input portion, the number of packets is divided into a predetermined number of divisions, and the control portion controls the powder supplying portion, the powder dividing portions, and the dispensing and packing portion such that the dispensing and packing is performed in order for each divided divisional unit.

Unfortunately, in the conventional medicine dispensing and packing apparatus, when an inputted large number of packets is divided into divisional units, a user such as a pharmacist needs to repetitively input and calculate the number of divisions until an appropriate number of divisions is obtained. Further, because such input work needs to be performed through the keyboard as the input portion, the operation is difficult for the user, and the input and the calculation are time-consuming and troublesome.

### CITATION LIST

### Patent Literature

Patent Literature 1: Japanese Patent Laid-Open No. 2007-97667

### SUMMARY

### Technical Problem

The present invention, which has been made in view of the above-mentioned circumstances, has an object to provide a medicine dispensing and packing apparatus that can simply perform dispensing and packing work without requiring a user such as a pharmacist to repetitively perform troublesome input and calculation.

### Solution to Problem

The present invention has been made in order to achieve the above-mentioned object, and a medicine dispensing and packing apparatus according to the present invention includes: a powder supplying portion that supplies powder; a plurality of powder dividing portions that divide the powder supplied from the powder supplying portion, into a plurality of portions by a predetermined dose; a dispensing and packing portion that dispenses and packs each dose of the powder divided by each of the plurality of powder dividing portions; a control portion that controls the powder supplying portion, the plurality of powder dividing portions, and the dispensing and packing portion; and an input portion through which prescription information is inputted to the control portion. The input portion includes an input screen to be operated by a user to input the prescription information. In a case where a number of packets larger than a maximum number of divisions by which one powder dividing portion can divide the powder and a dividing portion use instruction to dispense and pack the powder using the plurality of powder dividing portions are inputted as the prescription information to the control portion by operating the input screen, the control portion makes allocations of the inputted number of packets to the powder dividing portions within a range not exceeding the maximum number of divisions, and controls the powder supplying portion, the plurality of powder dividing portions, and the dispensing and packing portion on a basis of the allocations.

Further, in the medicine dispensing and packing apparatus according to the present invention, a number-of-packet input portion for enabling the user to input the number of packets may be displayed on the input screen. In a case where the number of packets larger than the maximum number of divisions is inputted by operating the number-of-packet input portion, a dividing portion use instruction input portion for enabling the user to input the dividing portion use instruction may be displayed in an operable state on the input screen. In a case where the dividing portion use instruction is inputted by operating the dividing portion use instruction input portion, the control portion may consecutively use the plurality of powder dividing portions to dispense and pack the powder, on the basis of the allocations.

Furthermore, in the medicine dispensing and packing apparatus according to the present invention, in a case where the number of packets and the dividing portion use instruction are inputted, a dividing portion order input portion for enabling the user to give an instruction on an order of use of the plurality of powder dividing portions may be displayed in an operable state on the input screen. The control portion may use the plurality of powder dividing portions one after another to dispense and pack the powder, on the basis of the order on which the instruction is given by operating the dividing portion order input portion.

Still further, in the medicine dispensing and packing apparatus according to the present invention, a plurality of allocation display portions for displaying the numbers of packets respectively allocated to the powder dividing portions may be displayed on the input screen so as to respectively correspond to the powder dividing portions.

Further, in the medicine dispensing and packing apparatus according to the present invention, the numbers of packets respectively displayed in the allocation display portions may be integers.

Furthermore, in the medicine dispensing and packing apparatus according to the present invention, the numbers of packets respectively displayed in the allocation display portions may be equal numbers.

### BRIEF DESCRIPTION OF DRAWINGS

FIG. 1 is a schematic view illustrating an embodiment of a medicine dispensing and packing apparatus according to the present invention.
FIG. 2 is a cross sectional view taken along a line A-A in FIG. 1.
FIG. 3 is a block diagram of the medicine dispensing and packing apparatus.
FIG. 4 is a diagram illustrating a menu screen of an input screen of the medicine dispensing and packing apparatus.
FIG. 5 is a diagram illustrating a usage input screen of the input screen of the medicine dispensing and packing apparatus.
FIG. 6 is a diagram illustrating a dispensing and packing information input screen of the input screen of the medicine dispensing and packing apparatus.
FIG. 7 is a diagram illustrating an operation screen of the input screen of the medicine dispensing and packing apparatus.
FIG. 8 is a diagram illustrating the operation screen of the input screen of the medicine dispensing and packing apparatus, the diagram illustrating a screen for selecting the order of use of a plurality of powder dividing portions.
FIG. 9 is a diagram illustrating the operation screen of the input screen of the medicine dispensing and packing apparatus, the diagram illustrating a screen for setting an allocation to each of the plurality of powder dividing portions.
FIG. 10 is a flow chart illustrating a procedure for inputting and setting prescription information concerning consecutive use in the medicine dispensing and packing apparatus.

### DESCRIPTION OF EMBODIMENTS

Hereinafter, an embodiment of a medicine dispensing and packing apparatus according to the present invention is described in detail with reference to the drawings.

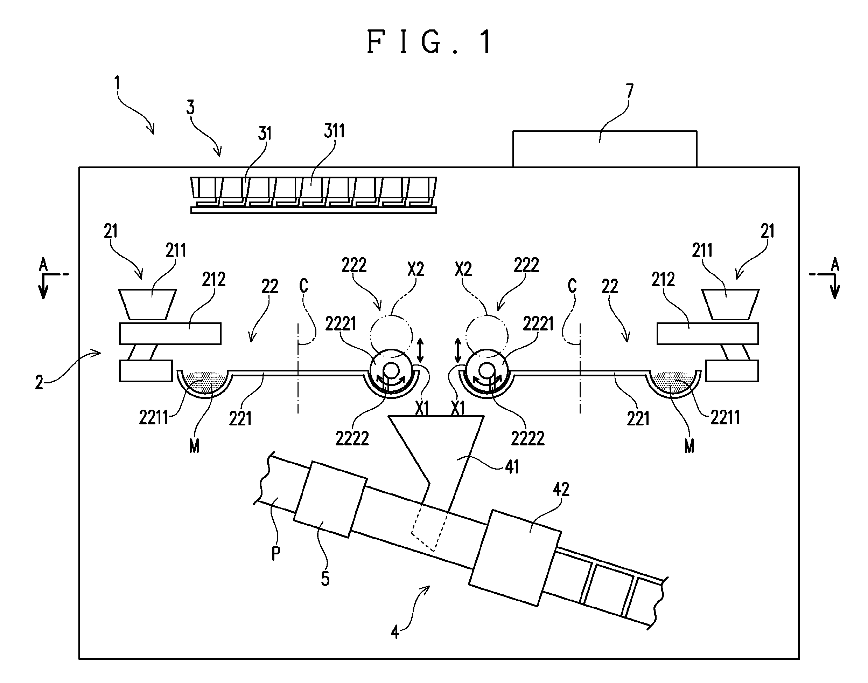

FIG. 1 illustrates a medicine dispensing and packing apparatus 1 according to the present embodiment. The medicine dispensing and packing apparatus 1 is an apparatus that can dispense and pack powder and tablets separately or together. The medicine dispensing and packing apparatus 1 includes: a powder distributing portion 2 used for powder; a tablet distributing portion 3 used for tablets; a dispensing and packing portion 4 that dispenses and packs powder M and tablets respectively distributed by the powder distributing portion 2 and the tablet distributing portion 3, separately or together; a printing portion 5 that performs printing on packing paper P used for the dispensing and packing; a control portion 6 that controls these portions; and an input portion 7 through which prescription information is inputted to the control portion 6.

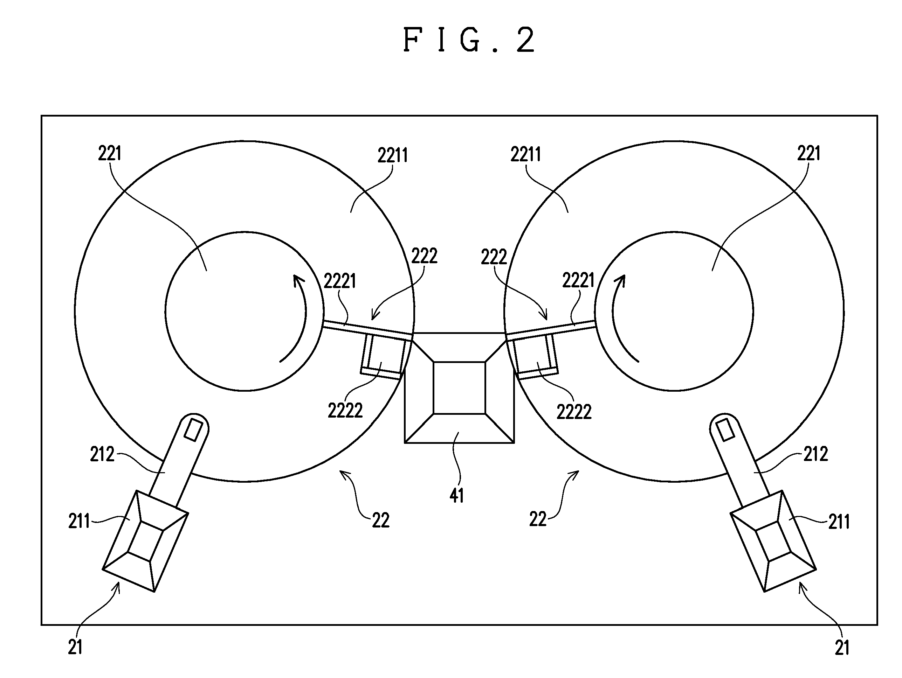

As illustrated in FIGS. 1 and 2, the powder distributing portion 2 includes: powder supplying portions 21 that supply the powder M; and powder dividing portions 22 that divide the powder M supplied from the powder supplying portions 21, into a plurality of portions by a predetermined dose.

The powder supplying portions 21 each include: a hopper 211 into which the powder M is put; and a feeder 212 that supplies the powder M put into the hopper 211, at a constant feeding speed. In the present embodiment, as illustrated in FIG. 2, the powder supplying portions 21 are respectively provided for the powder dividing portions 22 on a one-to-one basis, and can be used to respectively supply the powder M to the powder dividing portions 22.

The powder dividing portions 22 each include: a rotating table 221 on which the powder M supplied from each powder supplying portion 21 is mounted; and a scooper 222 that scoops the powder M on the rotating table 221 by a predetermined dose. The powder distributing portion 2 of the present embodiment includes the two powder dividing portions 22, 22 arranged side by side, and each powder dividing portion 22 can divide the powder M.

The rotating table 221 is a horizontally placed disc-shaped table, and is rotatable about a rotating axis C (the central axis of the disc shape) along the top-bottom direction. The peripheral portion of the rotating table 221 is provided with a concave portion 2211 opened upward. As illustrated in FIG. 1, the powder M supplied from each powder supplying portion 21 is put (deposited) in the concave portion 2211.

The scooper 222 includes: a stopper 2221 that stops the powder M put in the concave portion 2211 of the rotating table 221, by a predetermined angle about the rotating axis C; and a scooping blade 2222 that is driven so as to scoop the powder M stopped by the stopper 2221, outward in the radial direction of the rotating table 221. Further, on the basis of an external signal, the scooper 222 can be moved by a drive mechanism (not shown) between: a scooping position X1 at which the scooper 222 scoops the powder M put on the rotating table 221; and a retraction position X2 at which the scooper 222 is spaced apart from the rotating table 221. Note that the stopper 2221 abuts against the concave portion 2211 of the rotating table 221 at the scooping position X1, and is spaced apart from the concave portion 2211 thereof at the retraction position X2.

A distributing operation of the powder distributing portion 2 thus configured is described. First, the powder M is supplied from each powder supplying portion 21 to each powder dividing portion 22. Specifically, a user puts the powder M into the hopper 211. Then, the rotating table 221 is rotated about the rotating axis C at a constant speed. In this state, the feeder 212 is driven to supply a constant amount of the powder M to the rotating table 221. Consequently, the powder M is supplied from the feeder 212 to the concave portion 2211 of the rotating table 221, whereby a uniform amount of the powder M is put in the concave portion 2211 thereof in the circumferential direction.

Subsequently, each powder dividing portion 22 divides the powder M. Specifically, the scooper 222 is moved from the retraction position X2 to the scooping position X1, whereby the stopper 2221 abuts against the concave portion 2211. In this state, the rotating table 221 is rotated by a predetermined angle (for example, 18°). As a result, an amount of the powder M is stopped by the stopper 2221, the amount corresponding to the predetermined angle. Then, the scooping blade 2222 is driven to scoop the powder M stopped by the stopper 2221, outward in the radial direction from the concave portion 2211. In this way, the powder distributing portion 2 divides the powder M into a plurality of portions by a predetermined dose, and distributes each dose of the powder M to the dispensing and packing portion 4.

The tablet distributing portion 3 includes a tablet dividing and supplying portion 31 having 48 divisional cells 311. The divisional cells 311 are each provided with an openable and closable bottom plate. The bottom plates are designed to sequentially intermittently open above a chute (not shown) provided below the tablet dividing and supplying portion 31. With this design, the tablets in the divisional cells 311 are distributed from the chute to the dispensing and packing portion 4 via a route (not shown).

The dispensing and packing portion 4 includes: a common hopper 41 through which the powder M and the tablets respectively distributed by the powder distributing portion 2 and the tablet distributing portion 3 pass; and a heat sealer 42 that dispenses and packs each dose of the powder M and the tablets using the packing paper P. In the dispensing and packing portion 4, one dose of the powder M scooped from the concave portion 2211 of each rotating table 221 and one dose of the tablets ejected from each divisional cell 311 of the tablet dividing and supplying portion 31 are put onto the packing paper P through the common hopper 41, and are dispensed and packed by the heat sealer 42.

The printing portion 5 prints, for example, usage, patient information, doctor's directions, and the like on the surface of the packing paper P. The printing portion 5 is placed upstream of the common hopper 41 in the feeding direction of the packing paper P. A personal computer (to be described later) is used as the input portion 7 for outputting print items to the printing portion 5.

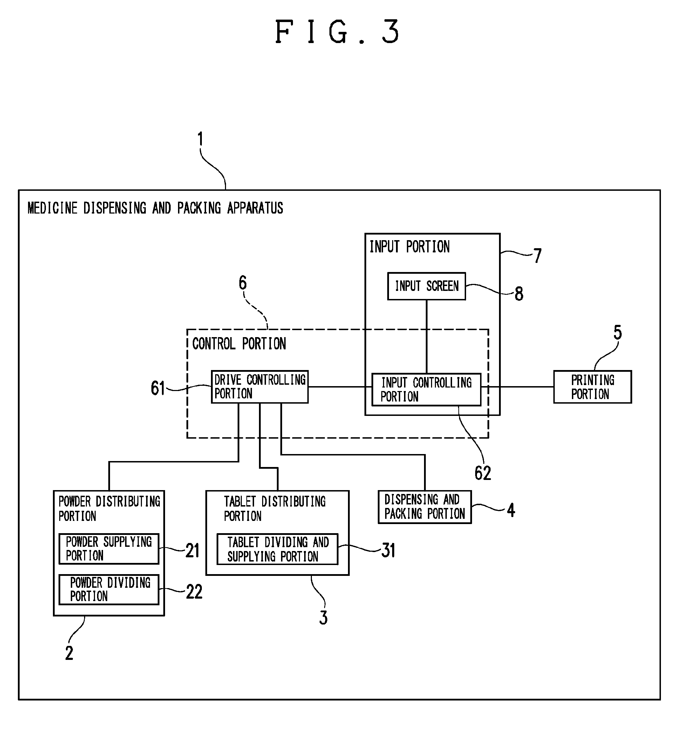

As illustrated in FIG. 3, the control portion 6 includes: a drive controlling portion 61 that controls the drive of the powder distributing portion 2, the tablet distributing portion 3, and the dispensing and packing portion 4; and an input controlling portion 62 that transmits prescription information to the drive controlling portion 61. The drive controlling portion 61 is a CPU built in the medicine dispensing and packing apparatus 1. The drive controlling portion 61 controls the drive of the powder distributing portion 2, the tablet distributing portion 3, and the dispensing and packing portion 4 on the basis of the inputted and set prescription information. The input controlling portion 62 is a CPU of an external personal computer connected to the medicine dispensing and packing apparatus 1.

Here, the personal computer functions as the input portion 7 for inputting the prescription information. Specifically, a touch panel is used for the input portion 7 (personal computer). That is, the input portion 7 (personal computer) includes: an input screen 8 (touch panel) to be operated by the user to input the prescription information; and the input controlling portion 62 (CPU) that transmits the inputted prescription information to the drive controlling portion 61. The prescription information inputted by a user's operation on the input screen 8 is checked and set by the drive controlling portion 61. The drive controlling portion 61 controls the respective portions on the basis of the settings. Further, the input controlling portion 62 controls the printing portion 5. Specifically, when print items are inputted on the input screen 8, the input controlling portion 62 controls the drive of the printing portion 5 on the basis of the inputted print items.

Here, the input screen 8 for inputting the prescription information is described.

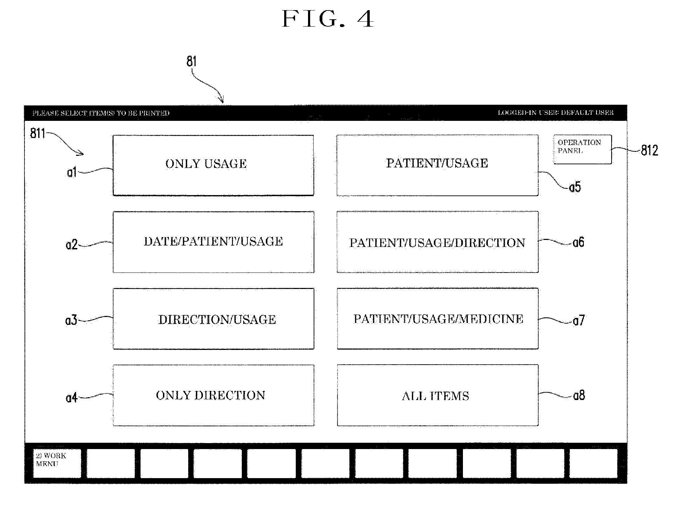

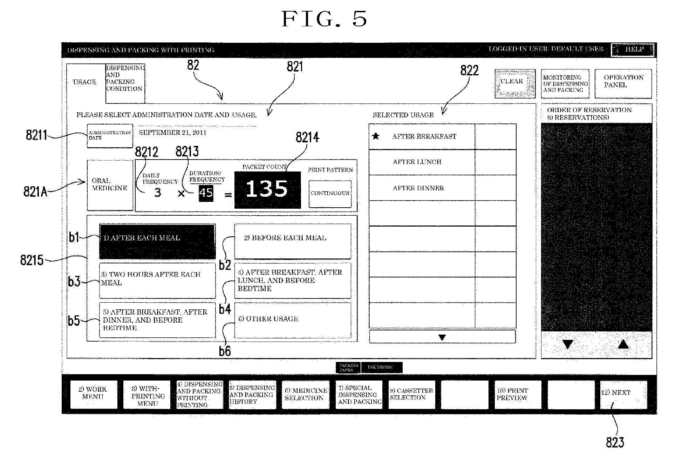

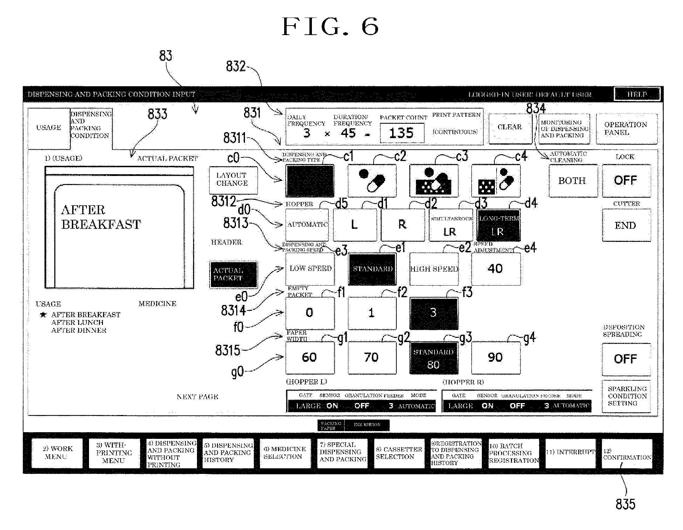

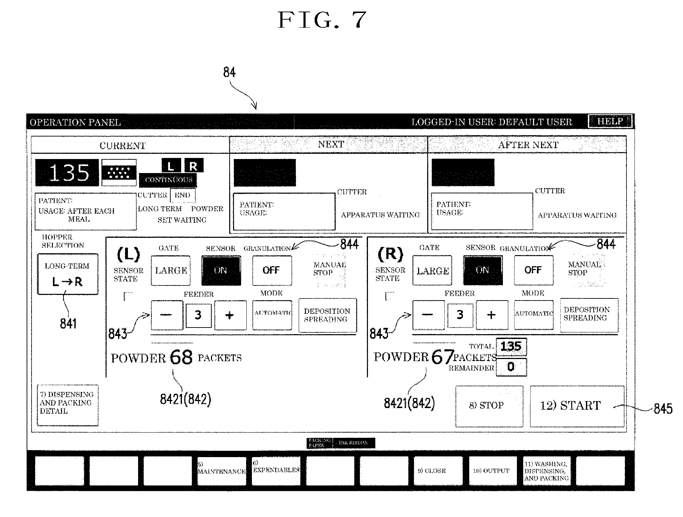

The input screen 8 is a touch-panel screen, and the selection of items to be printed on the packing paper P and the input of the prescription information can be performed by a user's touch operation on this screen. The input screen 8 is designed to be switchable among a print item selection screen 81 illustrated in FIG. 4, a usage input screen 82 illustrated in FIG. 5, a dispensing and packing information input screen 83 illustrated in FIG. 6, and an operation screen 84 illustrated in FIG. 7.

The print item selection screen 81 is a screen for selecting items to be printed on the packing paper P by the printing portion 5. The items to be printed on the packing paper P by the printing portion 5 are usage, a date, patient information, a medicine name, and directions. One or more or all of these items can be selected on the print item selection screen 81.

Specifically, as illustrated in FIG. 4, print item selection keys 811 respectively corresponding to a plurality of set print patterns are displayed as a print item selection portion on the print item selection screen 81. Specifically, the following keys are displayed as the print item selection keys 811 on the print item selection screen 81: a usage key a1 for printing only the usage; a date/patient/usage key a2 for printing the date, the patient information, and the usage; a direction/usage key a3 for printing the directions and the usage; a direction key a4 for printing only the directions; a patient/usage key a5 for printing the patient information and the usage; a patient/usage/direction key a6 for printing the patient information, the usage, and the directions; a patient/usage/medicine key a7 for printing the patient information, the usage, and the medicine name; and an all-item key a8 for printing all the items.

When a desired key of the print item selection keys 811 is selected on the print item selection screen 81 thus configured, the input controlling portion 62 sets item(s) corresponding to the selected key, as the print item(s), and outputs the set item(s) to the printing portion 5. In FIG. 4, the usage key a1 is selected. Note that the print item selection screen 81 includes an operation panel key 812 in addition to the print item selection keys 811, and is switched to the operation screen 84 when the operation panel key 812 is selected.

The usage input screen 82 is a screen for inputting usage information of the prescription information. Here, the usage information is information concerning the usage including: an administration date indicating the date of first administration; a daily frequency indicating the frequency of administration of one day; a duration/frequency indicating the number of days of administration; a packet count indicating the number of packets; and an administration timing indicating the period of time of administration. These pieces of information can be inputted and displayed on the usage input screen 82.

Specifically, as illustrated in FIG. 5, the following fields are displayed as a usage input portion 821 on the usage input screen 82: an administration date input field 8211 for inputting the administration date; a daily frequency input field 8212 for inputting the daily frequency; a duration/frequency input field 8213 for inputting the duration/frequency; a packet count display field 8214 for displaying the number of packets; and an administration timing input field 8215 for inputting the administration timing. When the user operates these fields, the usage information can be inputted and displayed on the usage input screen 82.

In the administration date input field 8211, the date of first administration can be inputted by a user's operation on a numeric keypad (not shown) or a calendar (not shown) displayed on the screen. In the daily frequency input field 8212 and the duration/frequency input field 8213, the frequency or the duration based on the contents of prescription can be inputted by a user's operation on a numeric keypad (not shown) displayed on the screen. In the administration timing input field 8215, timing keys respectively corresponding to a plurality of set administration timings are displayed. Specifically, the following keys are displayed as the timing keys in the administration timing input field 8215: an after-each-meal key b1; a before-each-meal key b2; a two-hour after-each-meal key b3; an after-breakfast, after-lunch, before-bedtime key b4; an after-breakfast, after-dinner, before-bedtime key b5; and an other-usage key b6. The user selects a key corresponding to a desired administration timing from among these keys such that the administration timing can be inputted.

Note that, when numerical values are respectively inputted to the daily frequency input field 8212 and the duration/frequency input field 8213, the total number of packets obtained by these numerical values is displayed in the packet count display field 8214. That is, in the present embodiment, a number-of-packet input portion 821A for enabling the user to input the number of packets includes the daily frequency input field 8212, the duration/frequency input field 8213, and the packet count display field 8214. When numerical values are respectively inputted to the daily frequency input field 8212 and the duration/frequency input field 8213, the total number of packets is calculated from these numerical values, and is displayed in the packet count display field 8214. Note that the number of packets may be directly inputted by a user's operation on the packet count display field 8214.

Further, an administration timing display field 822 for displaying a selected administration timing is displayed on the usage input screen 82. The administration timing display field 822 enables the user to check the contents of an administration timing inputted by his/her operation on the daily frequency input field 8212 and the administration timing input field 8215. Further, a next key 823 for proceeding to the next procedure is displayed on the usage input screen 82.

The dispensing and packing information input screen 83 is a screen for inputting dispensing and packing information of the prescription information. Here, the dispensing and packing information is information including: a dispensing and packing type for tablets and powder; a method of using the plurality of powder dividing portions 22; a dispensing and packing speed; the number of empty packets formed between the current dispensing and packing work and the next dispensing and packing work; and the width of the packing paper P used for the dispensing and packing. These pieces of information can be inputted and displayed on the dispensing and packing information input screen 83.

A plurality of dispensing and packing types, such as dispensing and packing of only powder, dispensing and packing of only tablets, simultaneous dispensing and packing (common packing) of tablets and powder, and individual dispensing and packing (separate packing) of tablets and powder, are set as the dispensing and packing type of the present embodiment. A plurality of use patterns, such as use of only any one of the two powder dividing portions 22, simultaneous use of the two, successive use of one and the other of the two (to be described later), and automatic use of the two, are set as the method of using the plurality of powder dividing portions 22. A high speed, a standard speed, a low speed, and an arbitrary speed are set as the dispensing and packing speed. 0 packets (no empty packet), 1 packet, and 3 packets are set as the number of empty packets. 60 mm, 70 mm, 80 mm (which is a standard paper width), and 90 mm are set as the paper width.

As illustrated in FIG. 6, a dispensing and packing information input portion 831 for enabling the user to input the dispensing and packing information is displayed on the dispensing and packing information input screen 83 for enabling the user to input the dispensing and packing information. The dispensing and packing information input portion 831 includes: a dispensing and packing type input portion 8311 for inputting the dispensing and packing type; a using method input portion 8312 for inputting the using method; a dispensing and packing speed input portion 8313 for inputting the dispensing and packing speed; an empty packet input portion 8314 for inputting an empty packet count; and a paper width input portion 8315 for inputting the paper width.

The dispensing and packing type input portion 8311 includes type selection keys c0 respectively corresponding to a plurality of practicable dispensing and packing types. Specifically, the dispensing and packing type input portion 8311 includes, as the type selection keys c0: a powder key c1 for setting the dispensing and packing type for only powder; a tablet key c2 for setting the dispensing and packing type for only tablets; a common packing key c3 for setting the simultaneous dispensing and packing; and a separate packing key c4 for setting the individual dispensing and packing. The user selects a key corresponding to a desired dispensing and packing type from among these keys such that the dispensing and packing type can be inputted.

The using method input portion 8312 includes method selection keys d0 respectively corresponding to a plurality of practicable using methods. Specifically, the using method input portion 8312 includes, as the method selection keys d0: a left use key d1 for using only one of the powder dividing portions 22; a right use key d2 for using only the other of the powder dividing portions 22; a simultaneous use key d3 for simultaneously using both the powder dividing portions 22; a consecutive use key d4 for consecutively using both the powder dividing portions 22; and an automatic use key d5 for automatically selecting the using method by the control portion 6. The user selects a key corresponding to a desired using method from among these keys such that the using method can be inputted. Note that, in the present embodiment, the consecutive use key d4 functions as a dividing portion use instruction input portion 7 for inputting an instruction to perform dispensing and packing using the plurality of powder dividing portions 22. The consecutive use key d4 is not normally displayed on the dispensing and packing information input screen 83, and is displayed in an operable state (selectable state) in the case where a large number of packets is inputted on the usage input screen 82.

The dispensing and packing speed input portion 8313 includes speed selection keys e0 respectively corresponding to a plurality of set dispensing and packing speeds. Specifically, the dispensing and packing speed input portion 8313 includes, as the speed selection keys e0: a standard key e1 for setting a standard dispensing and packing speed; a high-speed key e2 for setting a speed higher than the standard speed; and a low-speed key e3 for setting a speed lower than the standard speed. Further, the dispensing and packing speed input portion 8313 includes a speed input key e4 for inputting an arbitrary dispensing and packing speed.

The user selects any key of the standard key e1, the high-speed key e2, and the low-speed key e3 in the dispensing and packing speed input portion 8313 such that the dispensing and packing speed set correspondingly to the selected key can be inputted. Then, the user selects the speed input key e4 such that an arbitrary dispensing and packing speed can be inputted by a user's operation on a numeric keypad (not shown) displayed on the screen.

The empty packet input portion 8314 includes empty packet keys f0 respectively corresponding to a plurality of set empty packet counts. Specifically, the empty packet input portion 8314 includes, as the empty packet keys f0, a 0-packet key f1, a 1-packet key f2, and a 3-packet key f3. The user selects a key corresponding to a desired empty packet count from among these keys such that the empty packet count can be inputted.

The paper width input portion 8315 includes paper width keys g0 respectively corresponding to a plurality of set paper widths. Specifically, the paper width input portion 8315 includes, as the paper width keys g0, a 60 key g1, a 70 key g2, a 80 key g3 (standard key e1), and a 90 key g4. The user selects a key corresponding to the width of the packing paper P used for the dispensing and packing from among these keys such that the paper width can be inputted.

Further, the following portions and key are displayed on the dispensing and packing information input screen 83: a number-of-packet display portion 832 for displaying the number of packets; a print check portion 833 for checking contents to be printed on the packing paper P; a miscellaneous other-condition input portion 834 for inputting conditions concerning cleaning, locking, a cutter, and deposition spreading; and a confirmation key 835 for confirming the inputted usage information and the inputted dispensing and packing information.

Note that, in the present embodiment, the usage input screen 82 and the dispensing and packing information input screen 83 are displayed as tabs so as to overlap with each other within one screen. These input screens can be switched therebetween by selecting any of the tabs on the one screen.

The operation screen 84 is a screen for changing (inputting again) dispensing and packing conditions (for example, the method of using the plurality of powder dividing portions 22 and the numbers of packets to be dispensed and packed by the powder dividing portions 22) that have already been confirmed. Specifically, as illustrated in FIG. 7, the following portions are displayed on the operation screen 84: a using method change portion 841 for changing the method of using the plurality of powder dividing portions 22; and a number-of-packet change portion 842 for changing the numbers of packets to be dispensed and packed by the powder dividing portions 22 (in other words, the number of divisions of the powder dividing portions 22).

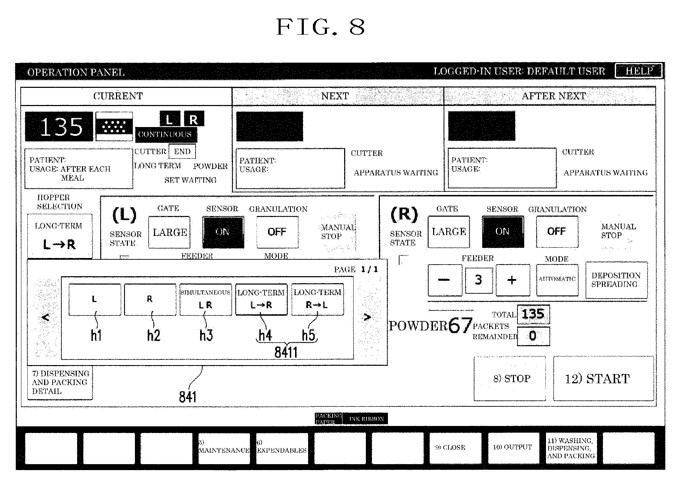

The using method change portion 841 serves to change (select again) the using method inputted through the using method input portion 8312. Hence, similarly to the using method input portion 8312, the using method change portion 841 includes a left use key h1, a right use key h2, and a simultaneous use key h3 (see FIG. 8). Further, the using method change portion 841 includes a dividing portion order input portion 8411 for changing (inputting) the order of use of the plurality of powder dividing portions 22 in the case of the consecutive use in which the plurality of powder dividing portions 22 are consecutively used. In the present embodiment, the dividing portion order input portion 8411 includes: a first order key h4 for first using the one of the two powder dividing portions 22 and then using the other thereof; and a second order key h5 for first using the other thereof and then using the one thereof. The dividing portion order input portion 8411 (the first order key h4 and the second order key h5) is not normally displayed on the operation screen 84, and is displayed in an operable state (selectable state) in the case where the consecutive use key d4 is selected in the using method input portion 8312 of the dispensing and packing information input screen 83 and where the consecutive use of the plurality of powder dividing portions 22 is inputted.

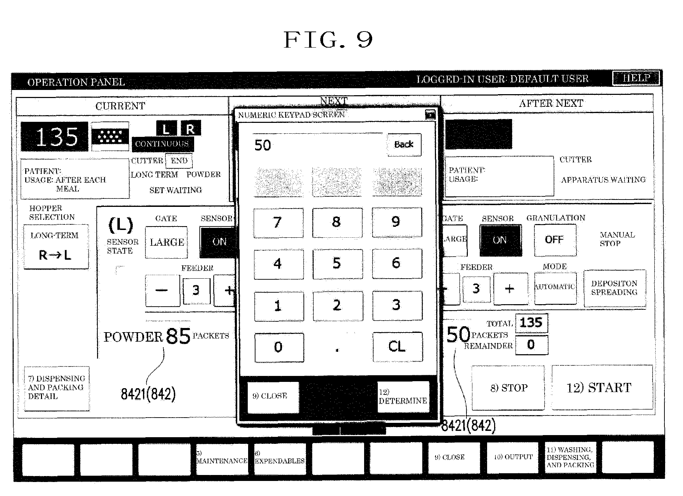

The number-of-packet change portion 842 serves to change the numbers of packets to be dispensed and packed by the powder dividing portions 22. The number-of-packet change portion 842 includes a plurality of packet count display portions 8421 for respectively displaying the numbers of packets of the powder dividing portions 22, the packet count display portions 8421 respectively corresponding to the powder dividing portions 22. In each packet count display portion 8421, as illustrated in FIG. 9, the number of packets to be dispensed and packed by the corresponding powder dividing portion 22 can be changed by a user's operation on a numeric keypad displayed on the operation screen 84. Further, in the case where the consecutive use is set, the packet count display portions 8421 respectively corresponding to the plurality of consecutively used powder dividing portions 22 are displayed in an operable state in the number-of-packet change portion 842. Moreover, when the user operates the packet count display portion 8421 corresponding to the one (or the other) of the powder dividing portions 22 to change the number of packets of the one (or the other) thereof, the number of packets of the packet count display portion 8421 corresponding to the other (or the one) of the powder dividing portions 22 is also changed on the basis of the contents of change and the total number of packets. Note that, in the case where the consecutive use is set, the packet count display portions 8421 that are provided so as to respectively correspond to the powder dividing portions 22 function as allocation display portions for displaying the numbers of packets respectively allocated to the plurality of consecutively used powder dividing portions 22.

Further, the following portions and key are displayed on the operation screen 84: a supply speed change portion 843 for changing the supply speeds of the powder supplying portions 21 (specifically, the feeders 212); a supply adjustment portion 844 for monitoring and adjusting supply conditions (deposition conditions) of the powder that is supplied from the powder supplying portions 21 to the powder dividing portions 22; and a start key 845 for starting dispensing and packing or completing a reservation thereof. Note that, in the present embodiment, not only the current dispensing and packing conditions but also the next reserved dispensing and packing conditions and the dispensing and packing conditions reserved after the next can be switchingly displayed and changed on the operation screen 84.

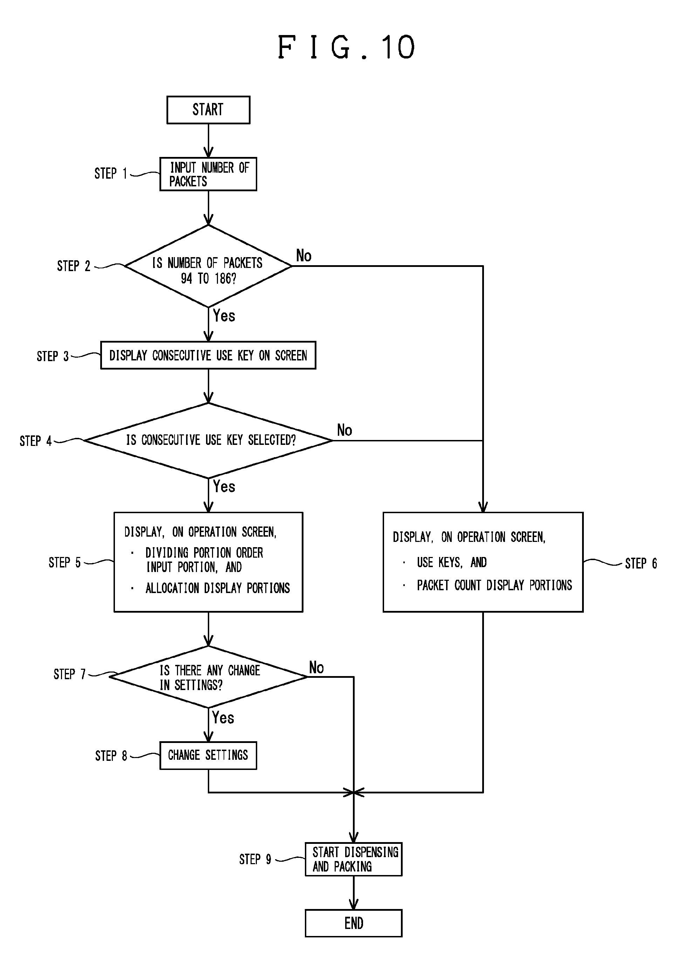

The medicine dispensing and packing apparatus 1 configured as described above can dispense and pack the tablets and the powder M. In particular, even in the case where a large number of packets that exceeds the maximum number of divisions by which one powder dividing portion 22 can divide the powder M is inputted, the medicine dispensing and packing apparatus 1 makes allocations of the large number of packets to the plurality of powder dividing portions 22, and consecutively uses the powder dividing portions 22, whereby the medicine dispensing and packing apparatus 1 can dispense and pack an amount of the powder M corresponding to the large number of packets, within one flow of dispensing and packing work. Here, a procedure for dispensing and packing the amount of the powder M corresponding to the large number of packets is described with reference to the input screen 8 illustrated in FIGS. 4 to 9 and a flow chart of FIG. 10. Note that the flow chart of FIG. 10 illustrates a procedure for an allocation to each powder dividing portion 22 in the case where the large number of packets is inputted.

First, as illustrated in FIG. 4, the user operates the print item selection screen 81 to select items to be printed on the packing paper P. In FIG. 4, because the usage key a1 is selected, the input controlling portion 62 controls the printing portion 5 to print only the usage. Upon the selection of the key, the print item selection screen 81 is switched to the usage input screen 82.

Subsequently, the user operates the input screen 8 to input the number of packets. The number of packets inputted in this case is a number larger than the maximum number of divisions (specifically, 93 packets) by which one powder dividing portion 22 can divide the powder M (Step 1 in FIG. 10). Specifically, the user operates the number-of-packet input portion 821A of the usage input screen 82 to input the number of packets in the current dispensing and packing work. In FIG. 5, the user inputs the frequency of administration of one day "3" to the daily frequency input field 8212, and inputs the number of days of administration "45" to the duration/frequency input field 8213. As a result, the total number of packets in the current dispensing and packing work is calculated, and "135" is displayed in the packet count display field 8214. Note that the date of first administration is inputted to the administration date input field 8211, and a predetermined timing key (in FIG. 5, the after-each-meal key b1) is selected in the administration timing input field 8215. Then, the next key 823 is selected so that the procedure goes to the next step.

Subsequently, when the number of packets is inputted, the control portion 6 determines whether or not the inputted number of packets is larger than the maximum number of divisions by which one powder dividing portion 22 can divide (dispense and pack) the powder M (Step 2 in FIG. 10). Specifically, when the next key 823 of the usage input screen 82 is selected, the input controlling portion 62 determines whether or not the inputted number of packets "135" is larger than the maximum number of divisions, and is equal to or less than the sum of the maximum numbers of divisions of the plurality of powder dividing portions 22. Along with this, the input controlling portion 62 switches the input screen 8 from the usage input screen 82 to the dispensing and packing information input screen 83. Note that, in the present embodiment, the maximum number of divisions is "93", and the sum of the maximum numbers of divisions is "186" because the number of the provided powder dividing portions 22 is two.

Here, in the case where the inputted number of packets is a value that is larger than the maximum number of divisions and is smaller than the sum of the maximum numbers of divisions of the plurality of powder dividing portions 22 (in the case of the present embodiment), as illustrated in FIG. 6, the input controlling portion 62 displays the consecutive use key d4 as the dividing portion use instruction input portion 7 in an operable state, in the dispensing and packing information input portion 831 of the dispensing and packing information input screen 83 (Step 3 in FIG. 10). In the case where the inputted number of packets is not such a value, the consecutive use key d4 is not displayed, the procedure proceeds to Step 6 in FIG. 10 upon the completion of the input of the dispensing and packing information, and the operation screen 84 is displayed.

Subsequently, the user operates the dispensing and packing information input screen 83 to input the dispensing and packing information (see FIG. 6). Specifically, the user selects one of the keys in the dispensing and packing type input portion 8311, in accordance with the dispensing and packing type to be adopted in the dispensing and packing work. In FIG. 6, the powder key c1 for setting the dispensing and packing type for only powder is selected. Then, the user selects one of the keys in the using method input portion 8312, in accordance with the method of using the plurality of powder dividing portions 22 to be adopted in the dispensing and packing work. Here, in the present embodiment, the plurality of powder dividing portions 22 are consecutively used to dispense and pack the amount of the powder M corresponding to the large number of packets. Hence, the consecutive use key d4 as the dividing portion use instruction input portion 7 is selected, whereby a dividing portion use instruction is inputted.

Note that one of the keys in each of the dispensing and packing speed input portion 8313, the empty packet input portion 8314, and the paper width input portion 8315 is selected, and miscellaneous other conditions are inputted by operating the miscellaneous other-condition input portion 834. Further, a print layout view corresponding to the print items selected on the print item selection screen 81 is displayed in the print check portion 833 of the dispensing and packing information input screen 83. Hence, the user can easily check the contents to be printed. Upon the completion of the input and check on the dispensing and packing information input screen 83, the confirmation key 835 is selected, and the inputted prescription information (the usage information and the dispensing and packing information) is transmitted from the input controlling portion 62 to the drive controlling portion 61.

Subsequently, upon the reception of the prescription information, the drive controlling portion 61 checks the contents of the received prescription information. Specifically, the drive controlling portion 61 determines whether or not the consecutive use key d4 is selected (Step 4 in FIG. 10). In the case where the consecutive use key d4 is selected, the drive controlling portion 61 instructs the input controlling portion 62 to display the first order key h4 and the second order key h5 as the dividing portion order input portion 8411 in an operable state, in the using method change portion 841 of the operation screen 84.

Here, a number of packets that exceeds the maximum number of divisions of one powder dividing portion 22 is inputted, and an instruction to perform dispensing and packing using the plurality of powder dividing portions 22 is given. Hence, the drive controlling portion 61 makes allocations of the inputted number of packets to the plurality of powder dividing portions 22. Specifically, the drive controlling portion 61 allocates a given number of packets to each powder dividing portion 22, within a range not exceeding the maximum number of divisions (in the present embodiment, "93") of one powder dividing portion 22. Note that the drive controlling portion 61 equally makes such allocations to the plurality of powder dividing portions 22. Specifically, in the case where the inputted number of packets is an even number, the drive controlling portion 61 allocates the same number of packets to each of the two powder dividing portions 22. In the case where the inputted number of packets is an odd number as in the present embodiment, the drive controlling portion 61 makes the number of packets allocated to the one of the powder dividing portions 22 larger by one than the number of packets allocated to the other of the powder dividing portions 22. Further, the drive controlling portion 61 sets the respective supply speeds of the powder supplying portions 21 to the same speed.

Further, the drive controlling portion 61 instructs the input controlling portion 62 to cause the packet count display portions 8421 respectively corresponding to the plurality of used powder dividing portions 22 to: function as the allocation display portions; and display, in an integer format, the numbers of packets respectively allocated to the powder dividing portions 22 in an operable state (Step 5 in FIG. 10). Note that, as described above, the drive controlling portion 61 equally makes allocations of the inputted number of packets to the plurality of powder dividing portions 22. Hence, the numbers of packets that are respectively displayed in the packet count display portions 8421, 8421 as the allocation display portions are integers and equal numbers (which are the same as each other in the case where the inputted number of packets is an even number, and are different from each other by one in the case where the inputted number of packets is an odd number).

On the other hand, in the case where the consecutive use key d4 is not selected, the drive controlling portion 61 does not display the dividing portion order input portion 8411, and displays only the use keys. Further, the drive controlling portion 61 instructs the input controlling portion 62 to cause the packet count display portions 8421 not to function as the allocation display portions but to function simply for the packet count display (Step 6 in FIG. 10). Note that the drive controlling portion 61 checks as appropriate other contents of the prescription information, and transmits the resultant check signals to the input controlling portion 62. As a result, the inputted prescription information is set to the medicine dispensing and packing apparatus 1, and the input screen 8 is switched from the dispensing and packing information input screen 83 to the operation screen 84.

Subsequently, the user operates the operation screen 84 to change the contents of settings as needed (Steps 7 and 8 in FIG. 10). In the present embodiment, in the case where the plurality of powder dividing portions 22 are consecutively used, the control portion 6 automatically sets the order of use of the plurality of powder dividing portions 22. Specifically, when a number of packets that exceeds the maximum number of divisions of one powder dividing portion 22 is inputted and when an instruction to perform dispensing and packing using the plurality of powder dividing portions 22 is given, the drive controlling portion 61 sets the order of use of the plurality of powder dividing portions 22 such that a powder dividing portion 22 different from the powder dividing portion 22 used in the dispensing and packing work immediately before the current dispensing and packing work is preferentially used. The automatically set order can be changed by a user's operation on the operation screen 84. Specifically, when the user operates the using method change portion 841 of the operation screen 84, the first order key h4 and the second order key h5 as the dividing portion order input portion 8411 are displayed as a window. When the user selects one of these keys, the order of use can be selected (changed) (see FIG. 8).

Further, when the user operates the number-of-packet change portion 842 of the operation screen 84, the numbers of packets respectively allocated to the plurality of powder dividing portions 22 can be changed. In the present embodiment, in the case where the plurality of powder dividing portions 22 are consecutively used, the control portion 6 equally makes allocations of the inputted number of packets to the plurality of powder dividing portions 22. Then, the allocated number of packets (integer) is displayed in the packet count display portion 8421 that functions as the allocation display portion of each powder dividing portion 22. In FIG. 7, "68" is displayed in the packet count display portion 8421 of the one of the powder dividing portions 22, and "67" is displayed in the packet count display portion 8421 of the other of the powder dividing portions 22. When the user operates the packet count display portions 8421 thus configured, the numbers of packets respectively allocated to the powder dividing portions 22 can be changed within a range not exceeding the maximum number of divisions. Specifically, as illustrated in FIG. 9, when the user operates each packet count display portion 8421 of the operation screen 84, a numeric keypad is displayed on the operation screen 84, and the allocated number of packets can be adjusted (changed) by a user's operation on the numeric keypad. Note that, in FIG. 9, the packet count display portion 8421 of the other of the powder dividing portions 22 is operated, and the number of packets of the other of the powder dividing portions 22 is changed from "67" to "50". Then, along with this change, the number of packets of the one of the powder dividing portions 22 is changed from "68" to "85".

When such a change in the contents of settings through a user's operation on the operation screen 84 is recognized by the input controlling portion 62 (Step 7 in FIG. 10), the contents of change are transmitted from the input controlling portion 62 to the drive controlling portion 61, and the drive controlling portion 61 changes the previously made settings (Step 8 in FIG. 10). Then, according to the display of the numbers of packets (specifically, the display of the packet count display portions 8421) respectively allocated to the powder dividing portions 22, the user weighs the powder M, and puts the weighed powder M into each hopper 211. After that, when the user selects the start key 845 of the operation screen 84, the dispensing and packing is started, or the dispensing and packing reservation is completed (Step 9 in FIG. 10). Note that, in the case where there is no change in the contents of settings, the dispensing and packing is started, or the dispensing and packing reservation is completed upon the selection of the start key 845 of the operation screen 84.

Note that, in the case where the inputted number of packets of tablets exceeds the maximum number of packets (48 packets) of the tablet dividing and supplying portion 31, the tablet dividing and supplying portion 31 is repetitively used until the dispensing and packing for the inputted number of packets is completed.

In the medicine dispensing and packing apparatus 1 configured as described above, when the user such as a pharmacist operates the usage input screen 82 to input, as the usage information, a number of packets larger than the maximum number of divisions by which one powder dividing portion 22 can divide the powder M and when the user operates the dispensing and packing information input screen 83 to input, as the dispensing and packing information, a dividing portion use instruction on the consecutive use of the plurality of powder dividing portions 22, the control portion 6 makes allocations of the inputted large number of packets to the powder dividing portions 22 within a range not exceeding the maximum number of divisions, and controls the powder supplying portions 21, the plurality of powder dividing portions 22, and the dispensing and packing portion 4 on the basis of the allocations. Accordingly, the user such as a pharmacist does not need to repetitively perform troublesome input and calculation, and can perform the dispensing and packing work through a simple screen operation.

Further, the usage input screen 82 is provided with the number-of-packet input portion 821A for inputting the number of packets, and, when the large number of packets is inputted by operating the number-of-packet input portion 821A, the consecutive use key d4 as the dividing portion use instruction input portion 7 is displayed in an operable state on the dispensing and packing information input screen 83. Hence, the user can operate the number-of-packet input portion 821A to input the number of packets on the usage input screen 82, and can operate the consecutive use key d4 to input the dividing portion use instruction on the dispensing and packing information input screen 83. Such input work is simple. Further, the consecutive use key d4, which is not normally displayed, is displayed in an operable state on the dispensing and packing information input screen 83 only in the case where the large number of packets is inputted on the usage input screen 82. Hence, an erroneous operation can be reliably prevented.

Furthermore, when the large number of packets is inputted on the usage input screen 82 and when the consecutive use key d4 is selected and the dividing portion use instruction is inputted on the dispensing and packing information input screen 83, the dividing portion order input portion 8411 for giving an instruction on the order of use of the plurality of powder dividing portions 22 is displayed in an operable state on the operation screen 84. Hence, the user can set the order of use of the plurality of powder dividing portions 22 through a simple panel operation. Further, because the powder dividing portions 22 are used according to the order, the dispensing and packing can be efficiently performed.

Still further, the operation screen 84 is provided with the packet count display portions 8421 that function as the allocation display portions so as to respectively correspond to the powder dividing portions 22, and hence the numbers of packets respectively allocated to the powder dividing portions 22 can be easily checked.

Further, the allocations of the large number of packets to the powder dividing portions 22 are equally made. Hence, loads on the powder dividing portions 22 can be distributed, and therefore the durability of the apparatus can be improved. Further, when the allocations are equally made, the amounts of powder to be respectively put into the hoppers 211 are also equal. Hence, the powder can be easily weighed, and the workload can be suppressed.

Furthermore, when the plurality of powder dividing portions 22 are consecutively used, a powder dividing portion 22 other than the powder dividing portion 22 used in the last dispensing and packing work is preferentially used. Hence, long-term continuous drive of one powder dividing portion 22 can be avoided, and therefore the durability of the apparatus can be improved. Further, when the powder M is supplied in advance from the powder supplying portion 21 to a powder dividing portion 22 that is not used in the last dispensing and packing work, the next dispensing and packing work (powder dividing work) can be started immediately after the end of the last dispensing and packing work, and the work efficiency can be improved. That is, a powder dividing portion 22 that is the one other than the powder dividing portion 22 used in the last dispensing and packing work and is ready for the dispensing and packing (ready for the powder supply from the powder supplying portion 21) is preferentially used for the work, whereby the work efficiency can be improved.

Hereinabove, an embodiment of the present invention has been described, but the present invention is not limited to the embodiment, and can be variously changed within a range not departing from the gist of the present invention.

For example, in the present embodiment, description is given of the case where the consecutive use key d4 as the dividing portion use instruction input portion 7 is not normally displayed, and is displayed in an operable state when the large number of packets is inputted by operating the number-of-packet input portion 821A, but the present invention is not limited thereto. The consecutive use key d4 may be normally displayed in an inoperable state, and the display thereof may be switched to an operable state when the large number of packets is inputted by operating the number-of-packet input portion 821A.

Further, in the present embodiment, description is given of the case where the dividing portion order input portion 8411 is not normally displayed, and is displayed in an operable state when the large number of packets and the dividing portion use instruction are inputted, but the present invention is not limited thereto. The dividing portion order input portion 8411 may be normally displayed in an inoperable state, and the display thereof may be switched to an operable state when the large number of packets and the dividing portion use instruction are inputted.

Furthermore, in the present embodiment, description is given of the case where the numbers of packets respectively allocated to the powder dividing portions 22 are equal, but the present invention is not limited thereto. The numbers of packets respectively allocated to the powder dividing portions 22 may be unequal. Specifically, a larger number of packets may be allocated to one powder dividing portion 22, and a smaller number of packets may be allocated to the other powder dividing portions 22. In this case, the numbers of packets that are integers and unequal numbers are respectively displayed in the corresponding allocation display portions.

Still further, in the present embodiment, description is given of the case where the control portion 6 is configured by two CPUs (the drive controlling portion 61 and the input controlling portion 62), but the present invention is not limited thereto. The control portion 6 may be configured by one CPU.

Further, in the present embodiment, description is given of the case where the number of the provided powder dividing portions 22 is two, but the present invention is not limited thereto. The number of the provided powder dividing portions 22 may be three or more. Note that, in the case where the number of the provided powder dividing portions 22 is two, the large number of packets is allocated to and dispensed and packed by all the powder dividing portions 22 (that is, the large number of packets is dispensed and packed using all the powder dividing portions 22), whereas, in the case where the number of the provided powder dividing portions 22 is three or more, the large number of packets is allocated to and dispensed and packed by at least two of the powder dividing portions 22 (that is, the large number of packets is dispensed and packed using at least two of the powder dividing portions 22).

Furthermore, in the present embodiment, description is given of the case where the plurality of powder dividing portions 22 are consecutively used, but the present invention is not limited thereto. The plurality of powder dividing portions 22 may be, for example, alternately used. Further, the medicine dispensing and packing apparatus 1 may be provided with an interrupt function, and dispensing and packing work for one consecutive use may be interrupted by different dispensing and packing work.

Further, in the present embodiment, the powder supplying portions 21 are respectively provided for the powder dividing portions 22 on a one-to-one basis, but the present invention is not limited thereto. One powder supplying portion 21 may be provided for the plurality of powder dividing portions 22.

The above description is summarized. The medicine dispensing and packing apparatus 1 according to the present embodiment includes: the powder supplying portions 21 that supply the powder M; the plurality of powder dividing portions 22 that divide the powder M supplied from the powder supplying portions 21, into a plurality of portions by a predetermined dose; the dispensing and packing portion 4 that dispenses and packs each dose of the powder M divided by each of the plurality of powder dividing portions 22; the control portion 6 that controls the powder supplying portions 21, the plurality of powder dividing portions 22, and the dispensing and packing portion 4; and the input portion 7 through which prescription information is inputted to the control portion 6. The input portion 7 includes the input screen 8 to be operated by the user to input the prescription information. In the case where a number of packets larger than the maximum number of divisions by which one powder dividing portion 22 can divide the powder M and a dividing portion use instruction to dispense and pack the powder M using the plurality of powder dividing portions 22 are inputted as the prescription information to the control portion 6 by operating the input screen 8, the control portion 6 makes allocations of the inputted number of packets to the powder dividing portions 22 within a range not exceeding the maximum number of divisions, and controls the powder supplying portions 21, the plurality of powder dividing portions 22, and the dispensing and packing portion 4 on the basis of the allocations.

In the medicine dispensing and packing apparatus 1 configured as described above, when the user such as a pharmacist operates the input screen 8 of the input portion 7 to input the number of packets and the dividing portion use instruction as the prescription information, the control portion 6 makes allocations of the inputted number of packets to the powder dividing portions 22 within a range not exceeding the maximum number of divisions. Then, the control portion 6 controls the powder supplying portions 21, the plurality of powder dividing portions 22, and the dispensing and packing portion 4 to perform dispensing and packing, on the basis of the allocations.

Further, in the medicine dispensing and packing apparatus 1, the number-of-packet input portion 821A for enabling the user to input the number of packets may be displayed on the input screen 8. In the case where the number of packets larger than the maximum number of divisions is inputted by operating the number-of-packet input portion 821A, the dividing portion use instruction input portion 7 for enabling the user to input the dividing portion use instruction may be displayed in an operable state on the input screen 8. In the case where the dividing portion use instruction is inputted by operating the dividing portion use instruction input portion 7, the control portion 6 may consecutively use the plurality of powder dividing portions 22 to dispense and pack the powder M, on the basis of the allocations.

According to such a configuration, the user can operate the number-of-packet input portion 821A to input the number of packets, and can operate the dividing portion use instruction input portion 7 to input the dividing portion use instruction. Hence, the input work of the user is simple. Further, in the case where the number of packets is inputted by operating the number-of-packet input portion 821A, the dividing portion use instruction input portion 7 is displayed in an operable state on the input screen 8. Hence, an erroneous operation of the user can be reliably prevented.

Furthermore, in the medicine dispensing and packing apparatus 1, in the case where the number of packets and the dividing portion use instruction are inputted, the dividing portion order input portion 8411 for enabling the user to give an instruction on the order of use of the plurality of powder dividing portions 22 may be displayed in an operable state on the input screen 8. The control portion 6 may use the plurality of powder dividing portions 22 one after another to dispense and pack the powder M, on the basis of the order on which the instruction is given by operating the dividing portion order input portion 8411.

According to such a configuration, the powder dividing portions 22 are used one after another. Hence, the dispensing and packing can be efficiently performed.

Still further, in the medicine dispensing and packing apparatus 1, the plurality of allocation display portions 8421 for displaying the numbers of packets respectively allocated to the powder dividing portions 22 may be displayed on the input screen 8 so as to respectively correspond to the powder dividing portions 22.

According to such a configuration, the display of the allocation display portions 8421 enables the user to check the numbers of packets respectively allocated to the powder dividing portions 22.

Further, in the medicine dispensing and packing apparatus 1, the numbers of packets respectively displayed in the allocation display portions 8421 may be integers.

Furthermore, in the medicine dispensing and packing apparatus 1, the numbers of packets respectively displayed in the allocation display portions 8421 may be equal numbers.

In this way, in the medicine dispensing and packing apparatus 1 according to the present embodiment, when the user such as a pharmacist operates the input screen 8 of the input portion 7 to input the number of packets and the dividing portion use instruction, the control portion 6 makes allocations of the inputted number of packets to the powder dividing portions 22 within a range not exceeding the maximum number of divisions. Then, the control portion 6 controls the powder supplying portions 21, the plurality of powder dividing portions 22, and the dispensing and packing portion 4 to perform dispensing and packing, on the basis of the allocations. Hence, the user does not need to repetitively perform troublesome input and calculation, and can perform the dispensing and packing work through a simple screen operation.

### REFERENCE SIGNS LIST

- 1: Medicine dispensing and packing apparatus
- 2: Powder distributing portion
- 3: Tablet distributing portion
- 4: Dispensing and packing portion
- 5: Printing portion
- 6: Control portion
- 7: Input portion (dividing portion use instruction input portion)
- 8: Input screen
- 21: Powder supplying portion
- 211: Hopper
- 212: Feeder
- 22: Powder dividing portion
- 221: Rotating table
- 222: Scooper
- 2211: Concave portion
- 2221: Stopper
- 2222: Scooping blade
- 31: Tablet dividing and supplying portion
- 311: Divisional cell
- 41: Common hopper
- 42: Heat sealer
- 61: Drive controlling portion
- 62: Input controlling portion
- 81: Print item selection screen
- 811: Print item selection key
- 812: Operation panel key
- 82: Usage input screen
- 821: Usage input portion
- 822: Administration timing display field
- 823: Next key
- 8211: Administration date input field
- 8212: Daily frequency input field
- 8213: Duration/frequency input field
- 8214: Packet count display field
- 8215: Administration timing input field
- 821A: Number-of-packet input portion
- 84: Operation screen
- 841: Using method change portion
- 842: Number-of-packet change portion
- 843: Supply speed change portion
- 844: Supply adjustment portion
- 845: Start key
- 8411: Dividing portion order input portion
- 8421: Packet count display portion (allocation display portion)
- P: Packing paper
- M: Powder

## Claims

1. A medicine dispensing and packing apparatus comprising:
a powder supplying portion that supplies powder;
a plurality of powder dividing portions that divide the powder supplied from the powder supplying portion, into a plurality of portions by a predetermined dose;
a dispensing and packing portion that dispenses and packs each dose of the powder divided by each of the plurality of powder dividing portions;
a control portion that controls the powder supplying portion, the plurality of powder dividing portions, and the dispensing and packing portion; and
an input portion through which prescription information is inputted to the control portion, wherein
the input portion includes an input screen to be operated by a user to input the prescription information, and
in a case where a number of packets larger than a maximum number of divisions by which one powder dividing portion can divide the powder and a dividing portion use instruction to dispense and pack the powder using the plurality of powder dividing portions are inputted as the prescription information to the control portion by operating the input screen, the control portion makes allocations of the inputted number of packets to the powder dividing portions within a range not exceeding the maximum number of divisions, and controls the powder supplying portion, the plurality of powder dividing portions, and the dispensing and packing portion on a basis of the allocations.

2. The medicine dispensing and packing apparatus according to claim 1, wherein
a number-of-packet input portion for enabling the user to input the number of packets is displayed on the input screen,
in a case where the number of packets larger than the maximum number of divisions is inputted by operating the number-of-packet input portion, a dividing portion use instruction input portion for enabling the user to input the dividing portion use instruction is displayed in an operable state on the input screen, and
in a case where the dividing portion use instruction is inputted by operating the dividing portion use instruction input portion, the control portion consecutively uses the plurality of powder dividing portions to dispense and pack the powder, on the basis of the allocations.

3. The medicine dispensing and packing apparatus according to claim 1 or 2, wherein
in a case where the number of packets and the dividing portion use instruction are inputted, a dividing portion order input portion for enabling the user to give an instruction on an order of use of the plurality of powder dividing portions is displayed in an operable state on the input screen, and
the control portion uses the plurality of powder dividing portions one after another to dispense and pack the powder, on the basis of the order on which the instruction is given by operating the dividing portion order input portion.

4. The medicine dispensing and packing apparatus according to claim 1 or 2, wherein a plurality of allocation display portions for displaying the numbers of packets respectively allocated to the powder dividing portions are displayed on the input screen so as to respectively correspond to the powder dividing portions.

5. The medicine dispensing and packing apparatus according to claim 3, wherein a plurality of allocation display portions for displaying the numbers of packets respectively allocated to the powder dividing portions are displayed on the input screen so as to respectively correspond to the powder dividing portions.

6. The medicine dispensing and packing apparatus according to claim 4, wherein the numbers of packets respectively displayed in the allocation display portions are integers.

7. The medicine dispensing and packing apparatus according to claim 6, wherein the numbers of packets respectively displayed in the allocation display portions are equal numbers.

8. The medicine dispensing and packing apparatus according to claim 5, wherein the numbers of packets respectively displayed in the allocation display portions are integers.

9. The medicine dispensing and packing apparatus according to claim 8, wherein the numbers of packets respectively displayed in the allocation display portions are equal numbers.
